# EUROPEAN PATENT APPLICATION

(11) **EP 4 831 637 A1**
(43) Date of publication of application: **28.10.2026**
(21) Application number: 26163894.4
(22) Date of filing: 11.03.2026
(51) Int. Cl.: G01N 33/57525

(54) **STAINING KIT AND METHOD FOR PREDICTING TREATMENT RESPONSE TO HEPATOCELLULAR CARCINOMA USING THE SAME**

(30) Priority: 24.04.2025 US 202519188634
(71) Applicant: Fullhope Biomedical Co., Ltd., New Taipei City 24159 (TW); Taipei Veterans General Hospital, Taipei City, Taiwan 11217 (TW)
(72) Inventor: Lee, Jan-Mou, 241 New Taipei City (TW); Fang, Chih-Hao, 241 New Taipei City (TW); Li, Po-Yu, 241 New Taipei City (TW); Chan, Chun-Wen, 241 New Taipei City (TW); Chu, Yen-Ying, 241 New Taipei City (TW); Lee, Cheng-Yun, 241 New Taipei City (TW); Chen, Yu-Hua, 241 New Taipei City (TW); Huang, Yi-Hsiang, 11217 Taipei City (TW); Lee, Pei-Chang, 11217 Taipei City (TW)
(74) Representative: Engelhard, Markus

(57) **Abstract**

A staining kit is provided, including antibodies against CD3, CD4, CD8, CD14, CD19, CD45, CD56, PD-1 and PD-L1, wherein the antibodies are each labeled with fluorescent dyes. A method of for predicting treatment response to hepatocellular carcinoma (HCC) in a subject using the staining kit is also provided.

## Description

### FIELD OF INVENTION

The present disclosure relates to a staining kit and uses thereof, and in particular to a method for predicting treatment response to hepatocellular carcinoma (HCC) in a subject using the staining kit.

### BACKGROUND OF THE INVENTION

Hepatocellular carcinoma (HCC) is the most common type of primary liver cancer, ranking as the sixth most prevalent cancer and the third leading cause of cancer-related mortality worldwide. More than half of HCC cases are diagnosed at an advanced stage, necessitating systemic therapy. In recent years, combination immunotherapy utilizing immune checkpoint inhibitors (ICIs) has emerged as a potential treatment strategy for unresectable HCC (uHCC). However, the objective response rate (ORR) to ICI-based immunotherapy remains limited, ranging from approximately 20% to 30%.

Although the phase III LEAP-002 clinical trial did not achieve its primary endpoint, the combination therapy of pembrolizumab and lenvatinib (PL) demonstrated comparable tumor responses and prolonged survival benefits in responders, particularly in real-world data from Asian populations. Despite these findings, an effective biomarker for predicting tumor response to combination immunotherapy has yet to be established, representing an unmet clinical need.

### SUMMARY OF THE INVENTION

In view of the description above, the present invention aims to develop a noninvasive and accurate method for identifying hepatocellular carcinoma (HCC) patients who may benefit from HCC treatment prior to treatment initiation.

Therefore, in one aspect, the present disclosure provides a method for predicting treatment response to HCC in a subject, including steps of:
(a) staining peripheral blood mononuclear cells (PBMCs) from the subject prior to HCC treatment using a staining kit, wherein the staining kit comprises antibodies against CD3, CD4, CD8, CD14, CD19, CD45, CD56, PD-1 and PD-L1, and the antibodies are each labeled with fluorescent dyes;
(b) performing biomedical data acquisition of fluorescent intensity of each antibody bound to characterized immune cell subsets related to the HCC using flow cytometry to obtain a dataset comprising data related to types of the characterized immune cell subsets and proportions thereof; and
(c) evaluating the dataset using an artificial intelligent (AI) model to predict whether the subject will exhibit a response or non-response to the HCC treatment.

In another aspect, the present disclosure provides a staining kit, including antibodies against CD3, CD4, CD8, CD14, CD19, CD45, CD56, PD-1 and PD-L1, wherein the antibodies are each labeled with fluorescent dyes.

Therefore, the present invention at least provides the following advantages:
1. The present invention discloses the differences in immune cell profiles (ICPs) between HCC patients who derive survival benefits from HCC treatment and those who do not.
2. The present invention identifies critical features in ICPs that significantly contribute to discriminating between ICPs from HCC patients who demonstrate and do not demonstrate survival benefits from HCC treatment.
3. The critical features identified by the present invention are useful for developing a noninvasive and accurate method for predicting which HCC patients are likely to benefit from HCC treatment prior to treatment initiation.
4. The staining kit of the claimed invention can be applied to easily and rapidly identify the characterized immune cell subsets related to the HCC.
5. The staining kit of the claimed invention can be utilized to efficiently and accurately predict HCC patients who may benefit from HCC treatment prior to treatment begins.

### BRIEF DESCRIPTION OF THE DRAWINGS

The present disclosure file contains at least one drawing executed in color. Copies of this patent or patent application publication with color drawing(s) will be provided by the Office upon request and payment of the necessary fee.
**FIG. 1** illustrates a flow chart of determining the characterized immune cell subsets related to HCC.
**FIG. 2** illustrates a study algorithm and patient selection according to an embodiment of the present invention.
**FIG. 3** illustrates the pedigree of peripheral immune cell subsets analyzed according to an embodiment of the present invention.
**FIG. 4** illustrates a flow chart of an AI model training developed using Python and trained with a machine learning algorithm using scikit-learn.
**FIG. 5** illustrates that the proportions of (**A**) monocytes, (**B**) natural killer (NK) cells, (**C**) natural killer T (NKT) cells, (**D**) total, (**E**) CD4⁺, and (**F**) CD8⁺ T cells in each group were compared using the Mann-Whitney U test. Data are presented as mean ± standard deviation in bar charts with individual data points. **p* < 0.05; ***p* < 0.01.
**FIGs. 6A-6C** respectively illustrate that the proportions of (**A**) regulatory, (**B**) PD-1⁺, and (C) PD-L1⁺ cells in each group were compared using the Mann-Whitney U test. Data are presented as mean ± standard deviation in bar charts with individual data points. **p* < 0.05.
**FIGs. 7A-7C** illustrate an AI model performance-ROC curve, wherein the trained AI model from a random forest algorithm combined with (**A**) Boruta, (**B**) Borutashap and (C) RFE feature selection methods via leave one out cross validation were acquired, and each area under curve (AUC) of the AI model is sequentially 1.0000, 0.7500, and 0.9167. The out of bag score from random forest is 0.7500.
**FIGs. 8A-8C** illustrate after AI model training from a random forest algorithm combined with (**A**) Boruta, (**B**) Borutashap and (**C**) RFE feature selection methods, the contribution of immune cell subsets in the discrimination was interpreted by a SHapley Additive exPlanations (SHAP) method.
**FIGs. 9A** **and** **9B** illustrate after model validation, the model testing results are summarized in a (**A**) confusion matrix and (**B**) classification report analysis.
**FIG. 10A** illustrates a SHAP force plot that breaks down the evaluation into individual feature contributions, offering an intuitive visualization of how each feature influences a single evaluation.
**FIG. 10B** illustrates a SHAP waterfall plot, showing how each feature contributes to the evaluation, highlighting their cumulative impact, where red represents a positive contribution to non-response evaluation, while blue indicates a negative contribution; the final evaluation is the sum of all contributions.

### DETAILED DESCRIPTION

### Definitions

The term "characterized immune cell subset" as used herein refers to that a group of immune cell subsets having a comparative immune profiling between responders who exhibit a response to HCC treatment and non-responders who exhibit non-response to HCC treatment, such as amounts of the immune cell subsets in the responders distinguishable from those in the non-responders.

The term "response" as used herein refers to a treatment outcome in which a subject exhibits an objective improvement, such as tumor shrinkage, a decrease in cancer cell burden, following HCC treatment.

The term "non-response" as used herein refers to a treatment outcome in which a subject exhibits HCC progression despite receiving HCC treatment.

The term "responder" as used herein refers to a subject exhibits a response to HCC treatment.

The term "non-responder" as used herein refers to a subject exhibits non-response to HCC treatment.

The term "hold-out set" as used herein is also called as "test set" and refers to the dataset not used in the training process of an AI model.

### Embodiments

In an embodiment, there is provided a method for predicting treatment response to hepatocellular carcinoma (HCC) in a subject, including steps of:
(a) staining peripheral blood mononuclear cells (PBMCs) from the subject prior to HCC treatment using a staining kit, wherein the staining kit comprises antibodies against CD3, CD4, CD8, CD14, CD19, CD45, CD56, PD-1 and PD-L1, and the antibodies are each labeled with fluorescent dyes;
(b) performing biomedical data acquisition of fluorescent intensity of each antibody bound to characterized immune cell subsets related to the HCC using flow cytometry to obtain a dataset comprising data related to types of the characterized immune cell subsets and proportions thereof; and
(c) evaluating the dataset using an artificial intelligent (AI) model to predict whether the subject will exhibit a response or non-response to the HCC treatment.

In an embodiment, the HCC treatment is a combination therapy. Preferably, the combination therapy includes pembrolizumab and lenvatinib.

In an embodiment, the use of the AI model includes applying a machine learning algorithm based on a random forest (RF) algorithm.

In an embodiment, said step (c) of evaluating the dataset comprises the following steps performed by the AI model:
generating a predicted probability derived from the machine learning algorithm, and
predicting the subject will exhibit the non-response to the HCC treatment when the predicted probability meets or exceeds a predefined threshold value; or predicting the subject will exhibit the response to the HCC treatment when the predicted probability fails to meet the predefined threshold value.

In an embodiment, the predefined threshold value of the predicted probability may be equal to 0.5-1.0. In an embodiment, the predefined threshold value may be 0.5, 0.55, 0.6, 0.65. 0.7, 0.75, 0.8, 0.85, 0.90, 0.95 or 1.0, preferably 0.5.

In an embodiment, the characterized immune cell subsets related to the HCC includes one, at least one, several or all, of CD8 T cell, PD-1⁺ CD8 NK cell, PD-L1⁺ monocyte, T cell, PD-L1⁺ CD8 T cell, and PD-1⁺ CD4 T cell.

In an embodiment, the characterized immune cell subsets related to the HCC may be identified by steps of:
(i) staining the PBMCs of a plurality of responders who exhibit a response to the HCC treatment and a plurality of non-responders who exhibit non-response to the HCC treatment, respectively, using a second staining kit (*e.g.,* as shown in **Table 3**);
(ii) performing data acquisition of fluorescent intensity of each antibody bound to the PBMCs of the responders and the non-responders, respectively, using flow cytometry;
(iii) identifying immune cell subsets in the PBMCs of the responders and the non-responders, respectively, using a pedigree method to obtain a dataset including data related to types of the immune cell subsets and proportions thereof;
(iv) performing data preprocessing of the dataset; and
(v) evaluating the preprocessed dataset using the AI model to obtain immune cell subsets of the responders distinguishable from those of the non-responders as the characterized immune cell subsets related to the HCC.

In an embodiment, the second staining kit comprises antibodies against CD3, CD4, CD8, CD14, CD19, CD25, CD45, CD56, FoxP3, PD-1 and PD-L1, and the antibodies are each labeled with fluorescent dyes.

In an embodiment, the step (v) of evaluating the preprocessed dataset includes the following steps performed by the AI model:
performing a feature selection from the preprocessed data using a Boruta, BorutaShap or RFE algorithm to obtain predetermined immune cell subsets related to the HCC; and
applying data of the predetermined immune cell subsets to train the AI model with a machine learning algorithm based on a random forest (RF) algorithm. Preferably, the method according to the present invention insofar as it relates to a staining of cells from a patient and the acquisition of biomedical data, e. g. flow cytometry data, is performed in-vitro and/or ex-vivo.

In a further aspect, the present invention also provides for a staining kit comprising antibodies against CD3, CD4, CD8, CD14, CD19, CD45, CD56, PD-1 and PD-L1, wherein the antibodies are each labelled with fluorescent dyes.

### Examples

### Example 1: Determining Characterized Immune Cell Subsets Related to HCC

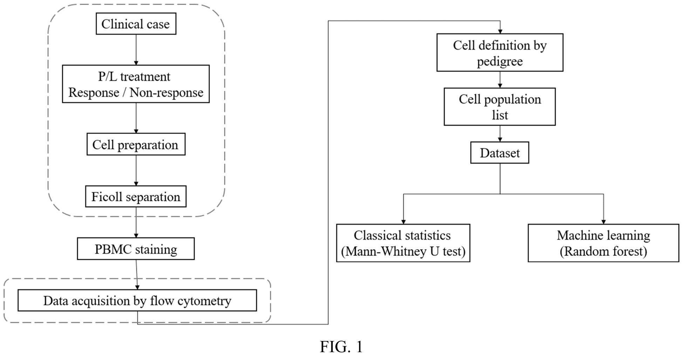

Please see **FIG. 1** for a flow chart of determining the characterized immune cell subsets related the HCC. The related steps thereof are described in detail below.

### Materials and Methods

### Patients, treatment, and outcome measurement

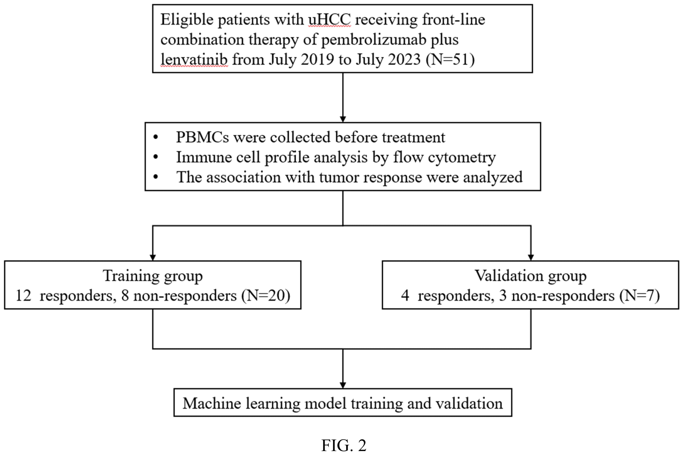

From July 2019 to July 2023, 51 patients who received front-line combination therapy of pembrolizumab plus lenvatinib for uHCC in Taipei Veterans General hospital were prospectively enrolled in a biomarker study prior to the initiation of ICI treatment. During this period, the ICIs approved by Taiwan Food and Drug Administration for HCC included nivolumab, pembrolizumab and atezolizumab. Unlike sorafenib or lenvatinib, the combination of atezolizumab and bevacizumab was not covered by the national health insurance until August 2023, leading to substantial out-of-pocket costs for patients. Consequently, the combination therapy of pembrolizumab and lenvatinib was prescribed for some patients following shared decision-making processes. Peripheral blood mononuclear cells (PBMCs) were collected from patients prior to the initiation of immunotherapy, provided they were free of active infection or had not taken antibiotics, antifungal drugs, steroids, non-steroidal anti-inflammatory drugs, or immunomodulatory agents within four weeks before starting ICI treatment. Immunophenotyping of PBMCs and its association with tumor response were analyzed. Subsequently, patients with radiologically confirmed objective response (i.e. responders, classified into R group) and those with primary progressive disease (non-responders, classified into NR group) were identified. The study algorithm is presented in **FIG. 2****.** The study was approved by the Institutional Review Board of Taipei Veterans General Hospital (IRB numbers: 2017-09-007CC, 2019-07-007AC, and 2019-08-006B).

Lenvatinib was administered according to the standard body weight (BW)-based recommendations: 12 mg/day for patients with a BW ≥ 60 kg or 8 mg/day for BW < 60 kg. Pembrolizumab was administered intravenously at a dose of 200 mg or 2-3 mg/kg every three weeks. Safety assessments and grading were performed using the National Cancer Institute Common Terminology Criteria for Adverse Events (NCI CTCAE, version 5.0). Tumor imaging was conducted by computed tomography or magnetic resonance imaging at screening and every 9 weeks during treatment. Tumor response was assessed using RECIST version 1.1 criteria based on contrast-enhanced radiologic images. Progressoin-free survival (PFS) was measured from the date of treatment initiation until tumor progression or death; while overall survival (OS) was measured from the date of starting treatment until death.

### Reagents and Antibodies

The reagents and antibodies used in this study are listed in **Tables 1 and 2.** All materials were aliquoted and stored under conditions recommended by the manufacturer.

**Table 1: Reagents**

| **Name** | **Manufacture** | **Catalogue number** |
|---|---|---|
| ***For PBMC and granulocyte isolation*** | | |
| Ficoll-Paque^{™} medium (Density 1.077 g/mL) | Cytiva | 17-5442-03 |
| FoxP3/Transcription Factor Staining Buffer Set | Thermo-Fisher | 00-5523-00 |
| Cell staining buffer | BioLegend | 420201 |

**Table 2: Antibodies and fluorescent dyes conjugated therewith**

| **Targets** | **Clone** | **Fluorophore** | **Manufacture** | **Cat. number** |
|---|---|---|---|---|
| CD3 | UCHT1 | BV510 | BioLegend | 300448 |
| CD4 | SFCI12T4D11 | PE-Cy7 | Beckman Coulter | 6607101 |
| CD8a | RPA-T8 | PB | BioLegend | 301023 |
| CD14 | RMO52 | APC-AF750 | Beckman Coulter | A86052 |
| CD19 | J3-119 | APC-AF750 | Beckman Coulter | A78838 |
| CD25 | 2A3 | BB515 | BD Pharmingen | 564467 |
| CD45 | J33 | ECD | Beckman Coulter | A07784 |
| CD56 | N901(NKH-1) | APC-AF700 | Beckman Coulter | B10822 |
| FoxP3 | 236A/E7 | APC | ebioscience | 17-4777-42 |
| PD-1 | EH12.2H7 | PerCP-Cy5.5 | BioLegend | 329914 |
| PD-L1 | 29E.2A3 | PE | BioLegend | 329706 |

| ***Isotype control*** | | | | |
|---|---|---|---|---|
| Mouse IgG1 κ | X40 | BB515 | BD Pharmingen | 564416 |
| Mouse IgG1 κ | MOPC-21 | PerCP-Cy5.5 | BioLegend | 400150 |
| Mouse IgG1 κ | P3.6.2.8.1 | APC | ebioscience | 17-4714-42 |
| Mouse IgG2b κ | MPC-11 | PE | BioLegend | 400312 |

| | | | | |
|---|---|---|---|---|
| * Abbreviations: AF700, Alexa Fluor 700; AF750, Alexa Fluor 750; APC, allophycocyanin; BB515, brilliant blue 515; BV510, brilliant violet 510; Cy5.5, cyanine 5.5; Cy7, cyanine 7; ECD, electron coupled dye; PB, pacific blue; PE, phycoerythrin; PerCP, peridinin-chlorophyll-protein. | | | | |

### Preparation of PBMCs

PBMCs were prepared following the manufacturer's protocol. Briefly, blood was prepared with PBS in a 1:1 dilution and separated by Ficoll-Paque (1.077 g/mL; Cytiva, Marlborough, MA, USA) density gradient centrifugation. PBMCs were collected from the buffy coat, suspended in 10% DMSO in PBS, and cryopreserved at -80°C until analysis.

### PBMC Immunostaining

Frozen PBMCs were thawed, and debris and dead cells were removed. Cells were sequentially stained with antibodies against surface and intracellular markers. In detail, PBMCs were sequentially stained with antibodies against cell surface and intracellular markers. All staining procedures were kept in dark. For surface marker staining, PBMCs were directly incubated with desired antibodies labeled with fluorescent dyes in the second staining kit (see **Tables 2 and 3**) for 10 minutes at 4°C. For intracellular marker staining, a part of surface marker-labeled PBMCs were fixed and permeabilized using FoxP3/Transcription Factor Staining Buffer Set (eBioscience^{™}) with recommended protocol from manual. Later, permeabilized PBMCs were stained with anti-FoxP3 antibody for 30 minutes at room temperature. Afterward, stained PBMCs were washed with staining buffer once and followed by analysis by flow cytometer.

**Table 3: Second staining kit**

| **Codes / Dyes** | **PT-129** |
|---|---|
| **FL1** | BB515-CD25 |
| **FL2** | PE-PD-L1 |
| **FL3** | ECD-CD45 |
| **FL4** | PerCP/Cy5.5-PD-1 |
| **FL5** | PE/Cy7-CD4 |
| **FL6** | APC-FoxP3 |
| **FL7** | APC-AF700-CD56 |
| **FL8** | APC-AF750-CD14 |
| | APC-AF750-CD19 |
| **FL9** | PB-CD8 |
| **FL10** | KO-CD3 |

### Data acquisition, pairwise comparison, and machine learning

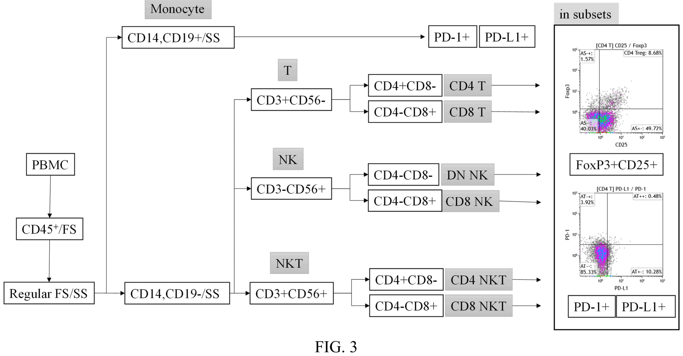

For data acquisition, the fluorescent intensity of PBMCs was analyzed using a Navios flow cytometer (Beckman Coulter, Brea, CA, USA), and fluorescence data were acquired and evaluated through Kaluza analysis software (version 1.3; Beckman Coulter). Using a pedigree method, a filtering approach with two-marker sets (parameters on the X-axis and Y-axis) was applied to define immune cell subsets so as to obtain a dataset of **FIG. 1****.** The definition of immune cell subsets is defined in **Table 4,** for which the filtration pedigrees are depicted in **FIG. 3****.** Because pembrolizumab is an anti-PD-1 antibody that inhibits the interaction of PD-1 with PD-L1, clinical responses to PL therapy can be determined based on the proportion of PD-1-related immune cells. Hence, a series of pedigrees for examining the proportion of T-cell and NK-cell lineages, including CD4⁺, CD8⁺, FoxP3⁺CD25⁺ (regulatory cells), and their PD-1⁺/PD-L1⁺ subpopulations were constructed. Notably, a study reported that patients with biliary cancer who did not demonstrate benefits from anti-PD-1 therapy had a higher proportion of circulating monocytes than those who demonstrated benefits from the therapy. Consequently, in this study, monocytes in this pedigree were included. Pairwise comparisons of the proportions of immune cell subsets, expressed as the proportions of each subset and the total CD45⁺ cells, were conducted between the R group and NR group.

**Table 4: Definition of immune cell subsets with markers**

| **Name of cell subsets** | **Markers** |
|---|---|
| ***Lineage cell*** | |
| Monocyte | CD14⁺CD19⁺ |
| T cell | CD3⁺CD14⁻CD19⁻CD56⁻ |
| CD4 T | CD3⁺CD14⁻CD19⁻CD56⁻CD4⁺CD8⁻ |
| CD8 T | CD3⁺CD14⁻CD19⁻CD56⁻CD4⁻CD8⁺ |

| ***NK cell*** | |
|---|---|
| NK | CD3⁻CD14⁻CD19⁻CD56⁺ |
| CD8 NK | CD3⁻CD14⁻CD19⁻CD56⁺CD4⁻CD8⁺ |
| DN NK | CD3⁻CD14⁻CD19⁻CD56⁺CD4⁻CD8⁻ |

| ***NKT cell*** | |
|---|---|
| NKT | CD3⁺CD14⁻CD19⁻CD56⁺ |
| CD4 NKT | CD3⁺CD14⁻CD19⁻CD56⁺CD4⁺CD8⁻ |
| CD8 NKT | CD3⁺CD14⁻CD19⁻CD56⁺CD4⁻CD8⁺ |

| **PD-1⁺ *cells*** | |
|---|---|
| PD-1⁺ NK | CD3⁻CD14⁻CD19⁻CD56⁺PD-1⁺PD-L1⁻ |
| PD-1⁺ CD8 NK | CD3⁻CD14⁻CD19⁻CD56⁺CD4⁻CD8⁺PD-1⁺PD-L1⁻ |
| PD-1⁺ DN NK | CD3⁻CD14⁻CD19⁻CD56⁺CD4⁻CD8⁻PD-1⁺PD-L1⁻ |
| PD-1⁺ CD4 NKT | CD3⁺CD14⁻CD19⁻CD56⁺CD4⁺CD8⁻PD-1⁺PD-L1⁻ |
| PD-1⁺ CD8 NKT | CD3⁺CD14⁻CD19⁻CD56⁺CD4⁻CD8⁺PD-1⁺PD-L1⁻ |
| PD-1⁺ monocyte | CD14⁺CD19⁺PD-1⁺PD-L1⁻ |
| PD-1⁺ CD4 T | CD3⁺CD14⁻CD19⁻CD56⁻CD4⁺CD8⁻PD-1⁺PD-L1⁻ |
| PD-1⁺ CD8 T | CD3⁺CD14⁻CD19⁻CD56⁻CD4⁻CD8⁺PD-1⁺PD-L1⁻ |

| ***PD-L1**⁺ **cells*** | |
|---|---|
| PD-L1⁺ NK | CD3⁻CD14⁻CD19⁻CD56⁺PD-1⁻PD-L1⁺ |
| PD-1⁺ CD8 NK | CD3⁻CD14⁻CD19⁻CD56⁺CD4⁻CD8⁺PD-1⁻PD-L1⁺ |
| PD-1⁺ DN NK | CD3⁻CD14⁻CD19⁻CD56⁺CD4⁻CD8⁻PD-1⁻PD-L1⁺ |
| PD-1⁺ CD4 NKT | CD3⁺CD14⁻CD19⁻CD56⁺CD4⁺CD8⁻PD-1⁻PD-L1⁺ |
| PD-1⁺ CD8 NKT | CD3⁺CD14⁻CD19⁻CD56⁺CD4⁻CD8⁺PD-1⁻PD-L1⁺ |
| PD-1⁺ monocyte | CD14⁺CD19⁺PD-1⁻D-L1⁺ |
| PD-1⁺ CD4 T | CD3⁺CD14⁻CD19⁻CD56⁻CD4⁺CD8⁻PD-1⁻PD-L1⁺ |
| PD-1⁺ CD8 T | CD3⁺CD14⁻CD19⁻CD56⁻CD4⁻CD8⁺PD-1⁻PD-L1⁺ |

| ***Regulatory cells*** | |
|---|---|
| CD4 T_{reg} | CD3⁺CD14⁻CD19⁻CD56⁻CD4⁺CD8⁻FoxP3⁺CD25⁺ |
| CD8 T_{reg} | CD3⁺CD14⁻CD19⁻CD56⁻CD4⁻CD8⁺FoxP3⁺CD25⁺ |
| CD8 NK_{reg} | CD3⁻CD14⁻CD19⁻CD56⁺CD4⁻CD8⁺FoxP3⁺CD25⁺ |
| DN NK_{reg} | CD3⁻CD14⁻CD19⁻CD56⁺CD4⁻CD8⁻FoxP3⁺CD25⁺ |
| CD4 NKT_{reg} | CD3⁺CD14⁻CD19⁻CD56⁺CD4⁺CD8⁻FoxP3⁺CD25⁺ |
| CD8 NKT_{reg} | CD3⁺CD14⁻CD19⁻CD56⁺CD4⁻CD8⁺FoxP3⁺CD25⁺ |

| | |
|---|---|
| *Abbreviations: DN, double negative; NK, natural killer cell; NKT, natural killer T cell; PD-1, programmed cell death 1; PD-L1, programmed cell death ligand 1; NK_{reg}; regulatory NK cell; T_{reg}, regulatory T cell. | |

This study adopted an AI model with a machine learning algorithm. Briefly, the data of patients were randomly divided into training and validation datasets (3:1 ratio). Immune cell subsets with highly correlated features (|Pearson's coefficient| ≥ 0.8) were excluded, and features were selected through recursive feature elimination. This model operates using a random forest (RF) algorithm with repeated stratified k-fold cross-validation, and Optuna is used for hyperparameter optimization. Receiver operating characteristic (ROC) analysis and a confusion matrix were used to evaluate the AI model. In addition, the contribution of each immune cell subset to ICPs was analyzed using SHapley Additive exPlanations (SHAP) method. The AI model was modified and implemented by scikit-learn under Python. All necessary tools for AI model building are available on GitHub.

### Statistical analysis

Continuous variables were expressed as the median (interquartile ranges [IQR]), while categorical variables were analyzed as frequencies and percentages. Pearson chi-square analyses or Fisher's exact tests were used to compare categorical variables, and the Mann-Whitney U tests or Kruskal-Wallis tests with Dunn's multiple comparisons were applied for continuous variables. The optimal cutoff values of bacterial abundance to predict tumor response were assessed using the area under receiver operating characteristic curves (AUC). Survival was estimated by the Kaplan-Meier method and compared by the log-rank test. Additionally, Cox's proportional-hazard model was used to identify prognostic factors for survival. To avoid the effect of collinearity, albumin-bilirubin grade and Child-Pugh class were not included in the same multivariate model. For all analyses, p < 0.05 was considered statistically significant. All statistical analyses were performed using the Statistical Package for Social Sciences (SPSS 26.0 for Windows, SPSS Inc, Chicago, IL, USA) and GraphPad Prism 9.5.1 (GraphPad Software, Huston, CA, USA).

### Classical Statistical Analysis for Determining Characterized Immune Cell Subsets

Classical statistics employs the Mann-Whitney U test, which is used to determine whether there is a difference in the medians between two independent samples. Through this method, it can be observed that immune system changes vary uniquely across different diseases, with statistically significant cell populations differing accordingly.

The chi-square test or Fisher exact test was used to compare categorical variables, and the Mann-Whitney U-test was used to compare numerical data. Statistical significance was set at p < 0.05. All analyses were performed using SPSS software (version 26.0, IBM SPSS Statistics for Windows; IBM, Armonk, New York, USA). The proportion of each immune cell is shown in a bar chart that was plotted using Prism version 9.5.1 (GraphPad Software Inc., La Jolla, CA, USA). The columns with statistical differences between the R and NR groups (p-values of the Mann-Whiteney-U test lower than 0.05, 0.01, 0.001, or 0.0001) are labeled with *, **, ***, or ****.

### AI Model Developed Using Python for Determining Characterized Immune Cell Subsets

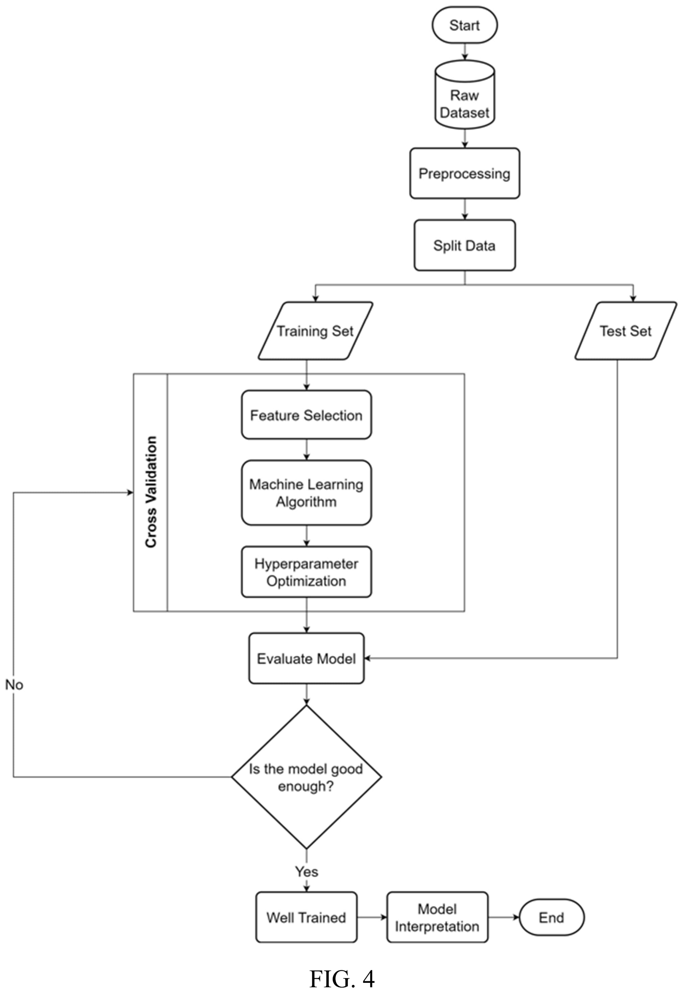

Please see **FIG. 4****,** which illustrates a flow chart of an AI model training developed using Python and trained with a machine learning algorithm using scikit-learn. In detail, the raw dataset described above is consisted of R group (i.e. responders, n = 16) and NR group (i.e. non-responders, n = 11). Data preprocessing was performed by the following steps. First, the target labels were converted to the numerical value 0 for R group and the numerical value 1 for NR group. 32 types of immune cell subsets were reserved for each subject after data preprocessing. The hold-out set (i.e. validation group of **FIG. 2** and test set of **FIG. 4**) consisted of some responders (n=4), which were split from the 16 responders, and some non-responders (n=3), which were split from the 11 non-responders. The remaining responders (n=12) and the remaining non-responders (n=8) were used as the training set (i.e. training group of **FIG. 2**). Feature selection in the training set was performed using Boruta, BorutaShap or RFE algorithm. The Boruta, BorutaShap or RFE algorithm was compared with the Z-scores of the shuffled shadow features and the original features to determine feature importance in every iteration. After the predefined iterations, predetermined immune cell subsets of the disease were obtained, which are more significantly relevant to classification than randomly permuted features.

The training set with the predetermined immune cell subsets was applied to train the AI model using a machine learning algorithm-Random forest (RF) algorithm, which was used to classify flow cytometry data from 2 classes of responders and non-responders.

The sensitivity and specificity of the trained AI model were then tested using the hold-out set. If the predicted probability of the trained AI model using the RF algorithm was greater than 0.5, the AI model predicted the subject as non-responders. The discriminative ability of the models was then evaluated by the area under curve (AUC) of the receiver operating characteristic (ROC) curve. The ROC curve is often used to compare the model performance in a clinical classification problem. It shows the relation of the true positive rate (sensitivity) against the false positive rate (1-specificity) for each possibility threshold. The better the discriminative ability of the AI model, the closer the ROC curve is to the upper left corner of the plot. Finally, the discriminative ability of the AI model was quantified by computing the area under the ROC curve using the trapezoidal rule to obtain the AUC results. The ROC curves and the AUC results of the training set were visualized to compare the AI model performance. The Shapley Additive exPlanations (SHAP) was applied to explain the AI model by computing the contribution of each predetermined immune cell subset to prediction. The SHAP summary plot was depicted to visualize the ranking of immune cell subset importance and the value of the feature per subject with the SHAP values. The color of the data point in each immune cell subset represents a high or a low feature value, in which each data point represents one subject. Red indicates the high value of immune cell subset, and blue indicates the low value of immune cell subset. The y-axis of the plot is the immune cell subset importance ranking of the predetermined immune cell subsets, and the x-axis is the SHAP value range. According to the trained AI model, the higher the SHAP value, the higher the subject are non-responders. Finally, the characterized immune cell subsets can be obtained via said AI model training.

### Results

### Demographics of participants

Participant characteristics are detailed in **Table 5.** The participants were predominantly male (76.5%), with a median age of 62.6 years, and the majority had chronic hepatitis B virus infection (60.8%). At baseline, most of the participants had Child-Pugh class A cancer (70.6%), although 60.8% had cancer classified as albumin-bilirubin grade >1. In addition, 45 patients (88.2%) exhibited Barcelona Clinic Liver Cancer stage C cancer. Following PL therapy, 16 participants exhibited objective responses, with a complete response in one subject and partial responses in 15 participants. In addition, 22 participants exhibited stable disease, and 11 participants exhibited progressive disease. 16 participants with objective responses and 11 with progressive diseases were stratified into the R group and the NR group, respectively. The two groups exhibited comparable liver function, incidence of portal vein invasion, extrahepatic metastasis, tumor characteristics, and serologic profiles.

**Table 5: Characteristics of enrolled subjects**

| | **Characteristics** | **All patients (N = 51)** | **Objective response (n = 16)** | **Progressive disease (n = 11)** | **P value** |
|---|---|---|---|---|---|
| Median age, y | | 62.6 (56.3-71.5) | 62.2 (57.2-67.5) | 65.7 (52.6-72.6) | 0.865 |
| Sex (male), n (%) | | 39 (76.5) | 10 (62.5) | 9 (81.8) | 0.405 |

| Viral infection | | | | | |
|---|---|---|---|---|---|
| | Non-viral HCC, n (%) | 13 (25.5) | 3 (18.8) | 3 (27.3) | |
| | HBsAg-positive, n (%) | 31 (60.8) | 9 (56.3) | 6 (54.5) | 0.838 |
| | Anti-HCV-positive, n (%) | 8 (15.7) | 4 (25.0) | 2 (18.2) | |

| Tumor burden | | | | | |
|---|---|---|---|---|---|
| | Max. tumor size, cm | 7.0 (3.0-10.0) | 4.5 (1.8-9.5) | 7.0 (5.0-10.0) | 0.481 |
| | Multiple tumors, n (%) | 36 (70.6) | 12 (75.0) | 6 (54.5) | 0.411 |
| | Extrahepatic metastasis, n (%) | 22 (56.4) | 8 (50.0) | 8 (72.7) | 0.427 |
| | Portal vein invasion, n (%) | 27 (52.9) | 7 (43.8) | 7 (63.6) | 0.440 |
| | Main PV invasion, n (%) | 13 (25.5) | 5 (31.3) | 4 (36.4) | 0.359 |
| | BCLC stage B/C, n (%) | 6/45 (11.8/88.2) | 11/5 (68.8/31.3) | 11/0 (100.0/0) | 0.060 |

| Serology | | | | | |
|---|---|---|---|---|---|
| | AFP, ng/mL | 185.0 (9.1-4974.0) | 225.6 (20.9-3229.3) | 242.0 (3.0-198260) | 0.942 |
| | AFP > 100 ng/mL, n (%) | 29 (56.9) | 9 (56.3) | 7 (63.6) | 1.000 |
| | CRP, mg/dL | 0.97 (0.28-3.02) | 0.73 (0.20-4.47) | 0.56 (0.30-1.10) | 0.904 |
| | ALT, U/L | 36 (20-70) | 20 (17-116) | 47 (31-75) | 0.231 |
| | AST, U/L | 76 (36-121) | 43 (21-118) | 97 (38-132) | 0.178 |
| | Total bilirubin, mg/dL | 0.97 (0.55-1.60) | 0.81 (0.56-1.84) | 1.00 (0.51-1.88) | 0.790 |
| | Albumin, g/dL | 3.7 (3.3-4.1) | 3.9 (3.3-4.2) | 3.9 (3.1-4.2) | 0.942 |
| | Creatinine, mg/dL | 0.84 (0.69-0.97) | 0.85 (0.70-0.93) | 0.76 (0.64-0.88) | 0.342 |

| Liver function | | | | | |
|---|---|---|---|---|---|
| | FIB-4 score | | | | |
| | Presence of ascites, n (%) | 19 (37.3) | 3 (18.8) | 4 (36.4) | 0.391 |
| | Child-Pugh score | 6 (5-7) | 5 (5-7) | 5 (5-6) | 0.942 |
| | Child-Pugh class A/B, n (%) | 36/15 (70.6/29.4) | 11/5 (68.8/31.2) | 9/2 (81.8/ 18.2) | 0.218 |
| | ALBI grade 1/2/3, n (%) | 20/29/2 (39.2/56.9/3.9) | 8/7/1 (50.0/43.8/6.3) | 4/7/0 (36.4/63.6/0) | 0.483 |

| Hematology | | | | | |
|---|---|---|---|---|---|
| | Prothrombin time, INR | 1.13 (1.04-1.26) | 1.11 (1.05-1.28) | 1.13 (1.04-1.26) | 0.981 |
| | Platelet count, K/cumm | 173 (118-219) | 170 (136-212) | 161 (107-251) | 0.827 |
| | White blood cell, cumm | 5600 (4400-7300) | 5460 (4900-7500) | 5300 (4200-5600) | 0.272 |
| | Neutrophil-lymphocyte ratio | 3.57 (2.35-5.58) | 2.97 (1.53-4.29) | 3.33 (1.92-3.98) | 0.645 |

| Clinical response to PL, n (%) | | | | | |
|---|---|---|---|---|---|
| | Complete response | 1 (2.0) | | | |
| | Partial response | 15 (29.4) | | | |
| | Stable disease | 22 (43.1) | | | |
| | Progressive disease | 11 (21.6) | | | |
| | Not assessable | 2 (3.9) | | | |
| | Objective response rate | 16 (31.4) | | | |
| | Disease control rate | 38 (74.5) | | | |

| | | | | | |
|---|---|---|---|---|---|
| *Abbreviations: *Abbreviation: Abbreviations: DN, double negative; NK, natural killer cell; NKT, natural killer T cell; PD-1, programmed cell death 1; PD-L1, programmed cell death ligand 1; NK_{reg}; regulatory NK cell; T_{reg}, regulatory T cell. | | | | | |

### Peripheral ICPs differ between R and NR groups (for classical statistics_Mann-Whitney U test)

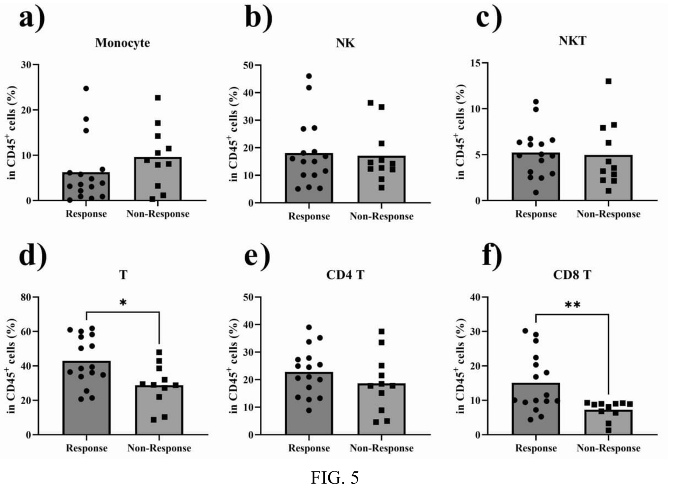

Please refer to **FIG. 5****.** Patients with disease progression after receiving the combination immunotherapy of pembrolizumab and lenvatinib (PL therapy) had an insufficient number of immune effector cells. Compared with the NR group, the R group exhibited significantly higher proportions of peripheral total T lymphocytes (42.88% vs. 28.78%, *p* = 0.0262) and CD8 T lymphocytes (15.09% vs. 7.27%, *p* = 0.0012). By contrast, the proportions of monocytes were marginally lower in the R group than the NR group (6.25% vs. 9.61%, *p* = 0.1341).

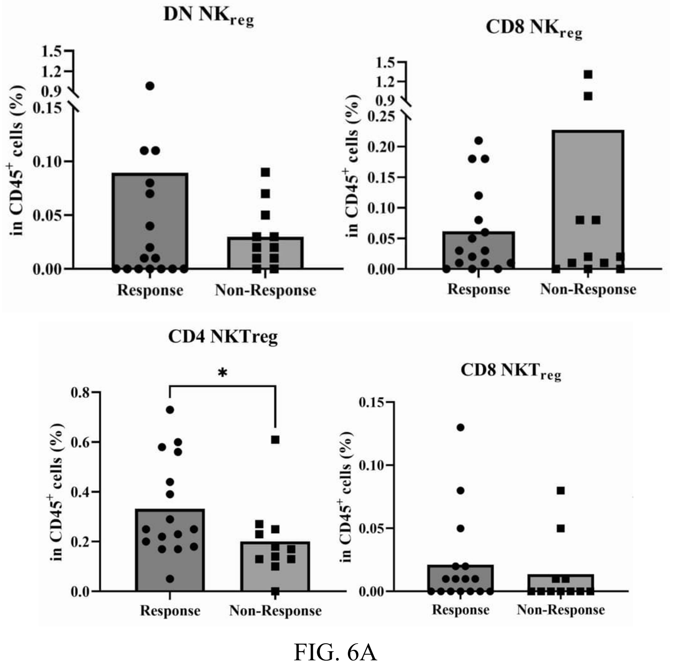

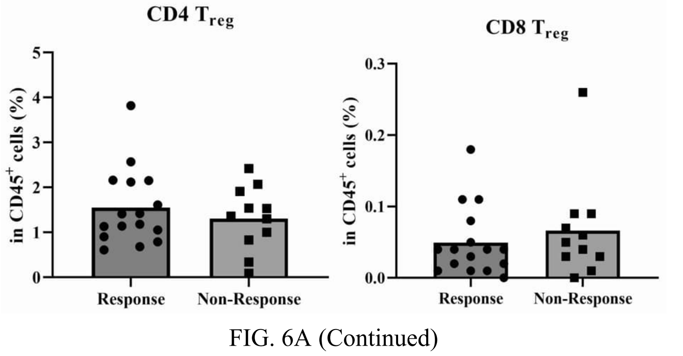

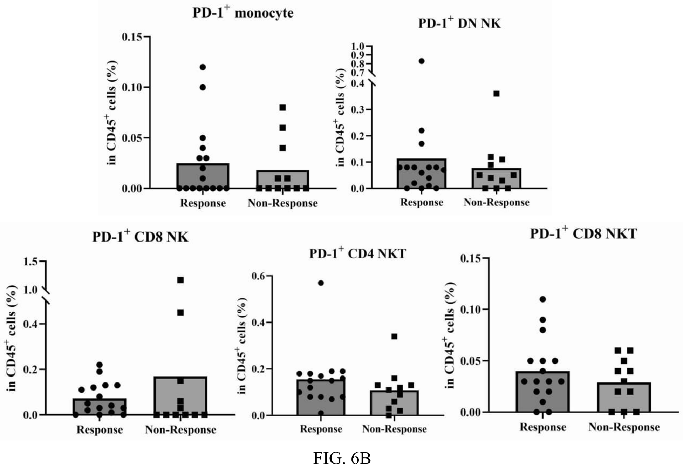

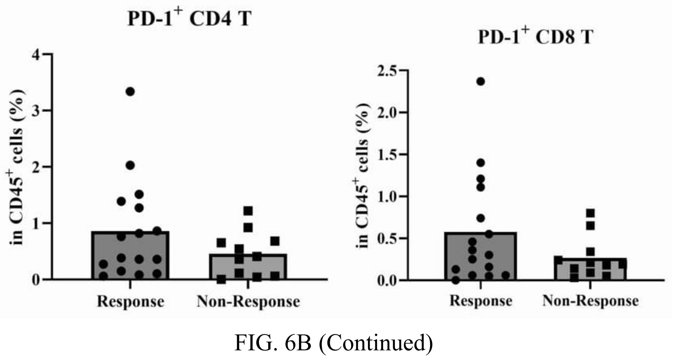

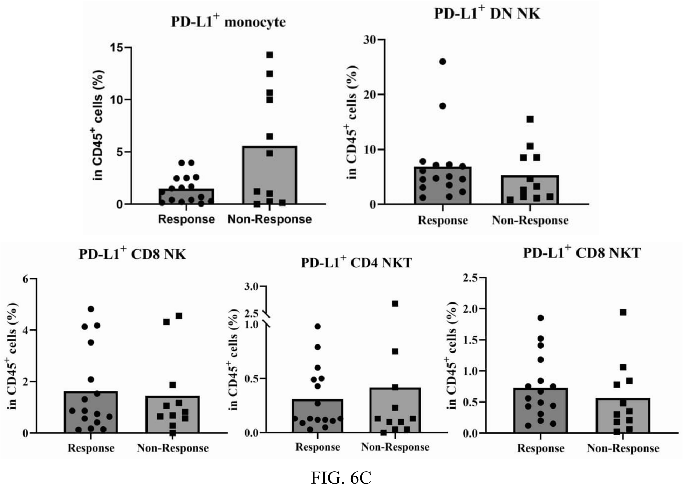

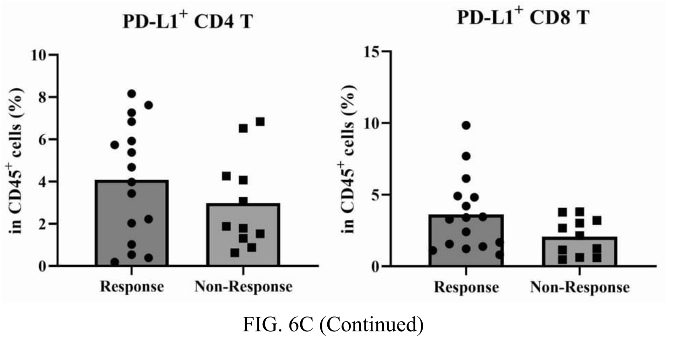

Regarding immune tolerance, patients who experienced disease progression after receiving the PL therapy exhibited reduced immune tolerance. Please refer to **FIGs. 6A-6C****.** The two response groups exhibited similar percentages of regulatory T cells, although a marginal decrease in CD4 NKT_{reg} cells was noted in the R and NR groups (**FIG. 6A**). Additionally, the R group exhibited higher proportions of PD-1⁺ CD4 T cells (0.86% vs. 0.45%, *p* = 0.2355, **FIG. 6B**) and PD-L1⁺ CD8 T cells (3.62% vs. 2.06%, *p* = 0.0797, **FIG. 6C**)) compared with the NR group. By contrast, the proportions of PD-1⁺ CD8 NK cells (0.07% vs. 0.17%, *p* = 0.3775, **FIG. 6B**) and PD-L1⁺ monocytes (1.48% vs. 5.58%, *p* = 0.1903, **FIG. 6C**) were higher in the NR group than in the R group.

### ICPs differ between R and NR groups (for AI model)

The AI model was utilized to evaluate the contribution of immune cell subsets to the separation of ICPs between the R and NR groups. The random forest (RF) machine learning model, combined with three feature selection algorithms-Boruta, BorutaShap, and RFE, was used to train the AI model using the same dataset.

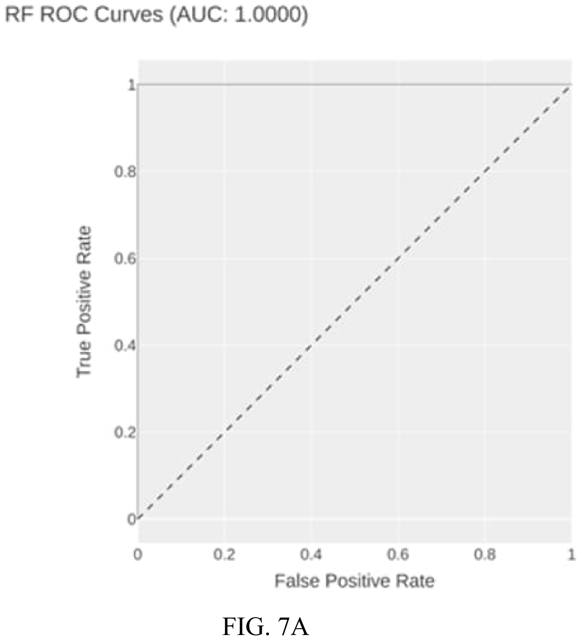

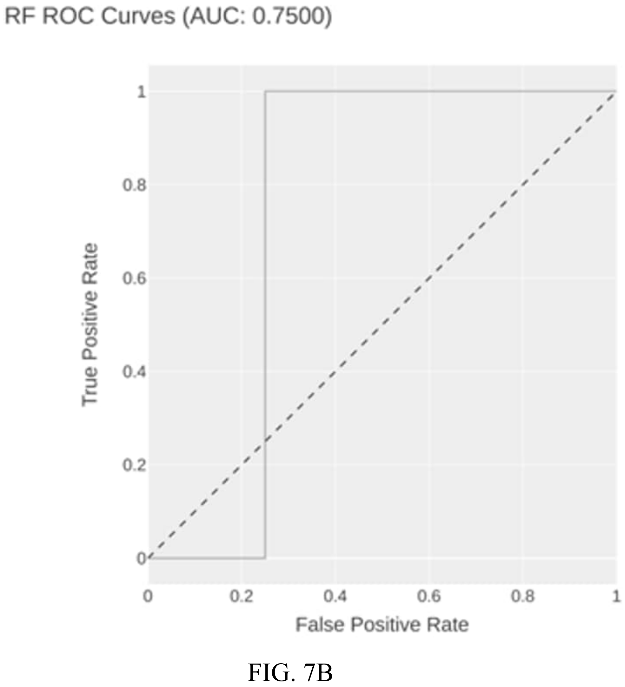

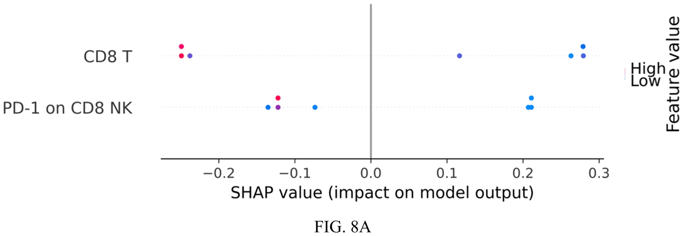

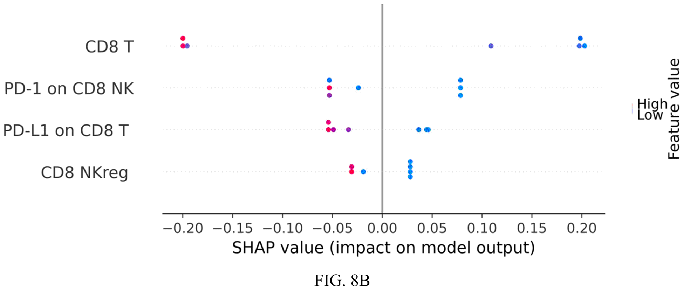

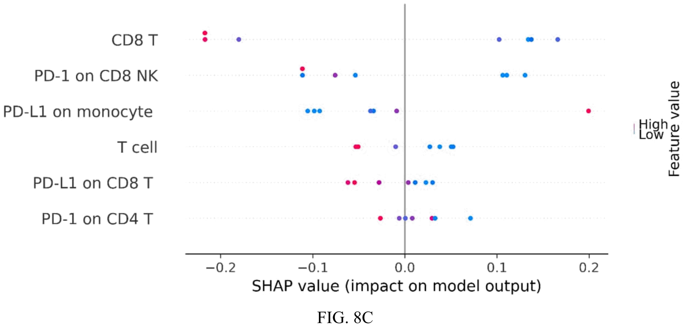

See **FIGs. 7A-7C**, **8A-8C** and **Table 6.** It was found that the AI model with RF-Boruta and RF-RFE algorithms exhibited superior performance in discriminating ICPs between the R and NR groups compared to AI models with RF-BorutaShap algorithm. Addtionally, the features selected by RF-RFE algorithm encompass all features selected by the RF-Boruta algorithm and RF-BorutaShap algorithms. Hence, RF-RFE algorithm was selected as the core algorithm of the AI model, and its six predetermined immune cell subsets (**FIG. 8C**) were determined as characterized immune cell subsets, as shown in **Table 7.**

**Table 6: Model performance- Sensitivity and Specificity of the hold-out set**

| **Algorithm** | **Sensitivity and Specificity of the hold-out set (%)** | |
|---|---|---|
| | NR group (n = 3) | R group (n = 4) |
| RF-Boruta | 100.0 | 75.0 |
| RF-BorutaShap | 67.0 | 75.0 |
| RF-RFE | 67.0 | 100.0 |

**Table 7: Characterized immune cell subsets related to HCC**

| **Characterized immune cell subsets** |
|---|
| CD8 T cell |
| PD-1⁺ CD8 NK cell |
| PD-L1⁺ monocyte |
| T cell |
| PD-L1⁺ CD8 T cell |
| PD-1⁺ CD4 T cell |

***The trained AI model accurately discriminates between R and NR groups in terms of their ICPs by use of RF-RFE algorithm***

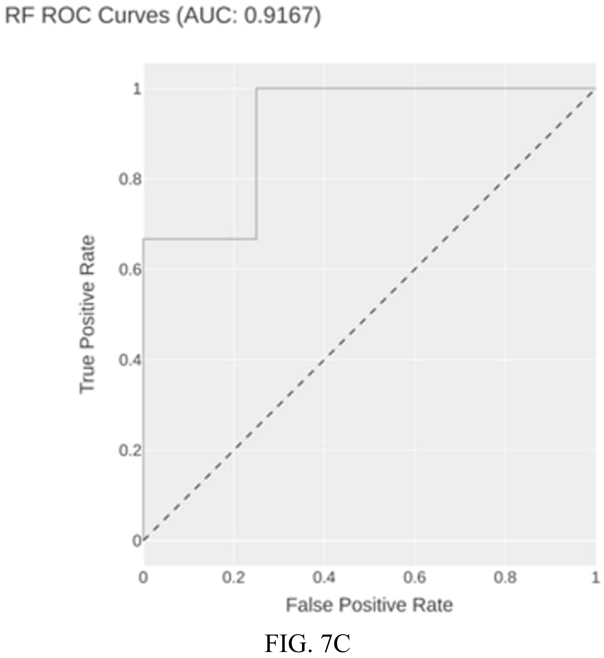

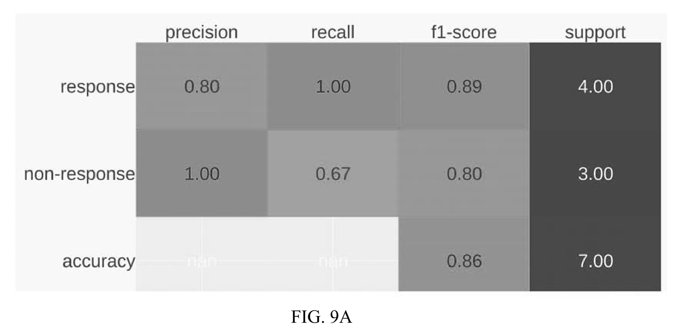

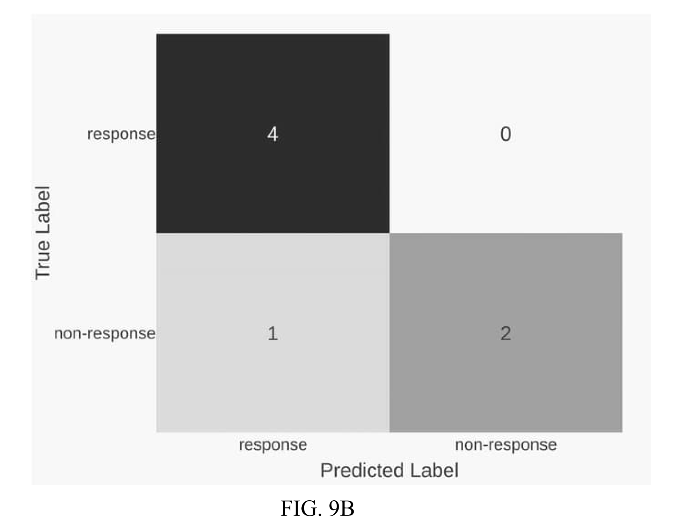

To determine the contribution of immune cell subsets to ICPs, the ICP data from the R and NR groups were used to train an AI model to differentiate responders from non-responders using their ICPs. Before model training, the ICP data were divided into training and validation datasets (3:1 ratio); these datasets were similar in terms of all demographic characteristics except gender (**Table 8**). After training, the contribution of each immune cell subset in the discrimination was evaluated by SHAP method. While applying validating dataset in testing the performance of the trained AI model, the trained AI model could correctly determine whether a patient was in the R (precision = 1.00) or NR (precision = 0.80) group on the basis of their ICP (**FIGs. 9A** **and** **9B**). The ROC analysis revealed an area under the curve (AUC) of 0.9167 (**FIG. 7C**). This AUC value indicated that the model could effectively differentiate between the ICPs of the R versus NR groups. In the SHAP analysis, six previously identified features were highly contributed to discrimination between ICPs from the R and NR groups as well. CD8 T cells exerted the most influence on the discrimination ability of the model (**FIG. 8C**). In summary, the AI model successfully discriminated between ICPs in the R versus NR groups; information on CD8 T cells was most instrumental to this discrimination.

**Table 8: Demographic of subjects in training and validation groups**

| | **Characteristics** | **Training group (N = 20)** | **Validation group (N = 7)** | **p value** |
|---|---|---|---|---|
| Median age, y (range) | | 60.1 (52.6-67.7) | 66.0 (62.3-72.6) | 0.800 |
| Sex (male), n (%) | | 17 (85.0) | 2 (28.6) | 0.011 |
| Viral infection | | | | |
| | Non-viral HCC, n (%) | 4 (20.0) | 2 (28.6) | |
| | HBsAg-positive, n (%) | 12 (60.0) | 3 (42.9) | 0.734 |
| | Anti-HCV-positive, n (%) | 4 (20.0) | 2 (28.6) | |
| Tumor burden | | | | |
| | Maximal tumor size, cm | 5.9 (1.5-10.0) | 8.2 (3.7-8.9) | 0.600 |
| | Multiple tumors, n (%) | 16 (80.0) | 5 (71.4) | 0.633 |
| | Extrahepatic metastasis, n (%) | 11 (55.0) | 5 (71.4) | 0.662 |
| | Portal vein invasion, n (%) | 9 (45.0) | 5 (71.4) | 0.385 |
| Serology | | | | |
| | AFP, ng/mL | 254.6 (6.5-4432.2) | 185.0 (16.8-582.0) | 0.100 |
| | AFP > 100 ng/mL, n (%) | 12 (60.0) | 4 (57.1) | 0.617 |
| | CRP, mg/dL | 0.73 (0.29-3.05) | 0.44 (0.30-0.46) | 0.800 |
| | ALT, U/L | 51 (18-117) | 34 (18-47) | 1.000 |
| | AST, U/L | 83 (31-125) | 49 (25-97) | 0.800 |
| | Total bilirubin, mg/dL | 0.74 (0.52-1.80) | 1.18 (0.61-1.88) | 1.000 |
| | Albumin, g/dL | 3.8 (3.3-4.2) | 4.0 (3.1-4.2) | 1.000 |
| | Creatinine, mg/dL | 0.83 (0.71-0.93) | 0.69 (0.62-0.90) | 0.600 |
| Liver function | | | | |
| | BCLC stage B/C, n (%) | 4/16 (20.0/800) | 1/6 (14.3/85.7) | 0.612 |
| | ALBI grade 1/2/3, n (%) | 10/9/1 (50.0/45.0/5.0) | 2/5/0 (28.6/71.4/0) | 0.513 |
| | Presence of ascites, n (%) | 5 (25.0) | 2 (28.6) | 0.703 |
| | Child-Pugh score | 5 (5-7) | 5 (5-6) | 0.600 |
| | Child-Pugh class A/B, n (%) | 14/6 (70.0/30.0) | 6/1/0 (85.7/14.3) | 0.077 |
| Hematology | | | | |
| | Prothrombin time, INR | 1.12 (1.04-1.27) | 1.24 (1.05-1.27) | 0.600 |
| | Platelet count, K/cumm | 173 (139-236) | 148 (55-213) | 0.200 |
| | White blood cell, cumm | 5400 (4900-7300) | 4710 ((3800-6500) | 0.200 |
| | Neutrophil-lymphocyte ratio | 2.92 (1.78-4.17) | 3.69 (1.90-6.22) | 0.990 |
| Tumor response | | | | |
| | Complete response | 1 (5.0) | 0 (0) | |
| | Partial response | 11 (55.0) | 4 (57.1) | |
| | Stable disease | 0 | 0 | 0.852 |
| | Progressive disease | 7 (35.0) | 3 (42.9) | |
| | Not assessable | 1 (5.0) | 0(0) | 0.852 |
| Objective response, n (%) | | 12 (60.0) | 4 (57.1) | 0.617 |

| | | | | |
|---|---|---|---|---|
| Abbreviation: AFP, α-fetoprotein; ALT, alanine aminotransferase; AST, aspartate aminotransferase; ALBI, albumin-bilirubin grade; BCLC, Barcelona Clinic Liver Cancer classification; CRP, C-reactive protein; HBsAg, anti-hepatitis B virus antibodies; HCC, hepatocellular carcinoma; HCV, hepatitis C virus; PL, pembrolizumab plus lenvatinib. | | | | |

Furthermore, the alignment between classical statistical analyses and the AI model in identifying relevant cell populations reinforces the validity and reliability of the AI-based selection. A substantial degree of concordance is observed, as the cell populations selected by the AI model largely overlap with those identified via conventional statistical criteria. Notably, the AI model selects 100% of the cell populations with statistical significance at p < 0.01, and 66.6% of those at p < 0.05. These findings suggest that the likelihood of selection by the AI model increases with the statistical significance of the cell populations, thereby supporting its capacity to prioritize biologically meaningful features.

### Example 2: Predicting Treatment Response to HCC in Subject Using Trained AI Model

PBMCs from a subject to be assessed was obtained for immunostaining. See the section "Preparation of PBMCs" and the section "PBMC Immunostaining" described above for details. The difference is that the PBMCs obtained the subject were stained via a staining kit, which includes antibodies against CD3, CD4, CD8, CD14, CD19, CD45, CD56, PD-1 and PD-L1, where the antibodies of each pattern are labeled with fluorescent dyes as shown in **Table 2.** Then, the biomedical data acquisition of the fluorescent intensities of antibodies bound to said 6 characterized immune cell subsets (see **Table 7**) was performed using flow cytometry to obtain a dataset that includes data related to types of the characterized immune cell subsets and proportions thereof.

The trained AI model evaluates the obtained dataset to generate a predicted probability derived from the RF machine learning algorithm combined with a RFE feature selection method. When the predicted probability of a dataset meets or exceeds the predefined threshold value of 0.5, it predicts the subject will exhibit the non-response to the HCC treatment. In this example, the predicted probability of the obtained dataset is 0.12, which fails to meet the threshold of 0.5, leading the AI model to predict this subject will exhibit the response to the HCC treatment.

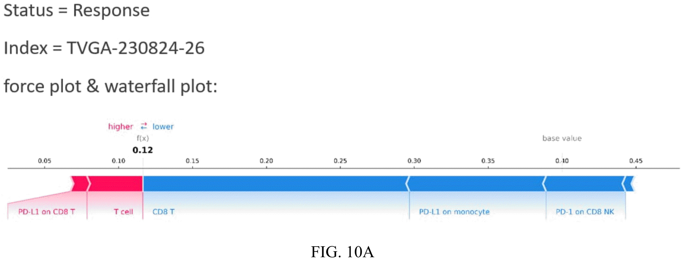

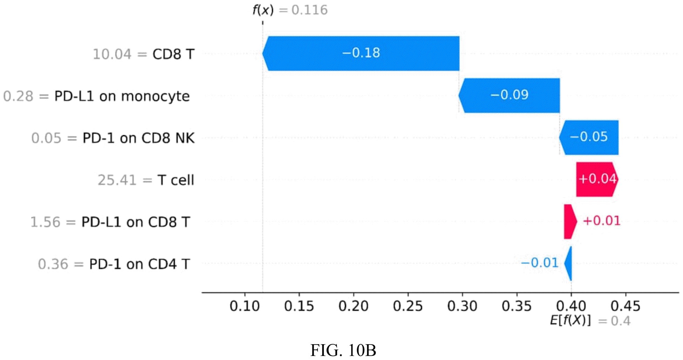

Refer to **FIGs. 10A** **and** **10B****.** The SHAP analysis was applied to break down each evaluation into feature contributions. The SHAP force plot offers an intuitive visualization of how individual features influence a single evaluation. The SHAP waterfall plot is another way to visualize individual evaluation, which provides a clearer view of how each feature contributes. The waterfall plot adds each feature's SHAP value sequentially and explicitly shows the stepwise accumulation of features effects. In this representation, red signifies a positive contribution toward the non-response evaluation, while blue indicates a negative contribution. The final evaluation is the sum of all these contributions. Both plots interpret model decisions more transparently by explaining how the model evaluates the contribution of each feature.

The CD8 T for the unknown data is 10.04, which is higher than the median of 8.74 in the non-response group and closely aligns with the median of 10.77 observed in the response group. The AI model identifies CD8 T as the most critical feature contributing to the classification of this unknown data into the response group. The T cell for the unknown data is 25.41, which is lower than the median of 38.94 in the response group and closely aligns with the average of 28.95 observed in the non-response group. The AI model identifies T cell as the most critical feature contributing to the classification of this unknown data into the non-response group. As illustrated in **FIGs. 10A** **and** **10B****,** within the feature contribution analysis of the unknown data, the negative contributions (represented in blue) exceed the positive contributions (represented in red). Consequently, the AI model aggregates these contributions and classifies this unknown data into a response group.

### Conclusion

Given that changes in the composition of circulating immune cell profiles (ICPs) may be associated with clinical responses to ICIs in patients with advanced cancer, these changes may be valuable for predicting the patients' clinical responses to combination immunotherapies. Our previous studies have demonstrated that circulating ICPs can be used to predict the efficacy of nivolumab monotherapy in patients with advanced HCC. In this study, we investigated the differences in circulating ICPs between patients with uHCC who achieved objective responses to PL therapy and those who did not. Additionally, we adopted a machine learning model that determines the contribution of each immune cell subset to ICPs.

Unless defined otherwise, all technical and scientific terms and any acronyms used herein have the same meanings as commonly understood by one of ordinary skill in the art in the field of this invention. Although any compositions, methods, kits, and means for communicating information similar or equivalent to those described herein can be used to practice this invention, the preferred compositions, methods, kits, and means for communicating information are described herein.

All references cited herein are incorporated herein by reference to the full extent allowed by law. The discussion of those references is intended merely to summarize the assertions made by their authors. No admission is made that any reference (or a portion of any reference) is relevant prior art. Applicants reserve the right to challenge the accuracy and pertinence of any cited reference.

## Claims

1. A method for predicting treatment response to hepatocellular carcinoma (HCC) in a subject, comprising steps of:
(a) staining peripheral blood mononuclear cells (PBMCs) from the subject prior to HCC treatment using a staining kit, wherein the staining kit comprises antibodies against CD3, CD4, CD8, CD14, CD19, CD45, CD56, PD-1 and PD-L1, and the antibodies are each labeled with fluorescent dyes;
(b) performing biomedical data acquisition of fluorescent intensity of each antibody bound to characterized immune cell subsets related to the HCC using flow cytometry to obtain a dataset comprising data related to types of the characterized immune cell subsets and proportions thereof; and
(c) evaluating the dataset using an artificial intelligent (AI) model to predict whether the subject will exhibit a response or non-response to the HCC treatment.

2. The method of claim 1, wherein the HCC treatment is a combination therapy.

3. The method of claim 2, wherein the combination therapy comprises pembrolizumab and lenvatinib.

4. The method of any one of claims 1-3, wherein the use of the AI model comprises applying a machine learning algorithm based on a random forest (RF) algorithm.

5. The method of any one of claims 1-4, wherein the step (c) of evaluating the dataset comprises the following steps performed by the AI model:
generating a predicted probability derived from the machine learning algorithm, and
predicting the subject will exhibit the non-response to the HCC treatment when the predicted probability meets or exceeds a predefined threshold value; or predicting the subject will exhibit the response to the HCC treatment when the predicted probability fails to meet the predefined threshold value.

6. The method of any one of claims 1-5, wherein the characterized immune cell subsets related to the HCC comprises CD8 T cell, PD-1⁺ CD8 NK cell, PD-L1⁺ monocyte, T cell, PD-L1⁺ CD8 T cell, and PD-1⁺ CD4 T cell.

7. The method of any one of claims 1-6, wherein the characterized immune cell subsets related to the HCC are identified by steps of:
(i) staining the PBMCs of a plurality of responders who exhibit a response to the HCC treatment and a plurality of non-responders who exhibit non-response to the HCC treatment, respectively, using a second staining kit;
(ii) performing data acquisition of fluorescent intensity of each antibody bound to the PBMCs of the responders and the non-responders, respectively, using flow cytometry;
(iii) identifying immune cell subsets in the PBMCs of the responders and the non-responders, respectively, using a pedigree method to obtain a dataset comprising data related to types of the immune cell subsets and proportions thereof;
(iv) performing data preprocessing of the dataset; and
(v) evaluating the preprocessed dataset using the AI model to obtain immune cell subsets of the responders distinguishable from those of the non-responders as the characterized immune cell subsets related to the HCC.

8. The method of claim 7, wherein the second staining kit comprises antibodies against CD3, CD4, CD8, CD14, CD19, CD25, CD45, CD56, FoxP3, PD-1 and PD-L1, and the antibodies are each labeled with fluorescent dyes.

9. The method of claim 7 or 8, wherein the step (v) of evaluating the preprocessed dataset comprises the following steps performed by the AI model:
performing a feature selection from the preprocessed data using a Boruta, BorutaShap or RFE algorithm to obtain predetermined immune cell subsets related to the HCC; and
applying data of the predetermined immune cell subsets to train the AI model with a machine learning algorithm based on a random forest (RF) algorithm.

10. A staining kit, comprising antibodies against CD3, CD4, CD8, CD14, CD19, CD45, CD56, PD-1 and PD-L1, wherein the antibodies are each labeled with fluorescent dyes.
